(19) Europäisches Patentamt
European Patent Office
Office européen des brevets

(11) EP 4 477 169 A1

(12) EUROPEAN PATENT APPLICATION

(43) Date of publication:
18.12.2024 Bulletin 2024/51

(21) Application number: 24180700.7

(22) Date of filing: 07.06.2024

(51) International Patent Classification (IPC):
*A61B 18/12* (2006.01)

(52) Cooperative Patent Classification (CPC):
**A61B 18/1206;** A61B 18/1233; A61B 18/1477;
A61B 2018/00517; A61B 2018/00547;
A61B 2018/00601; A61B 2018/00642;
A61B 2018/00767; A61B 2018/00827;
A61B 2018/1213

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 14.06.2023 US 202363472838 P

(71) Applicant: Olympus Winter & Ibe GmbH
22045 Hamburg (DE)

(72) Inventors:
• **Janich, Fabian**
**14469 Potsdam (DE)**
• **Dijkstra, Jelle**
**12205 Berlin (DE)**

(74) Representative: **Eisenführ Speiser**
**Patentanwälte Rechtsanwälte PartGmbB**
**Postfach 10 60 78**
**28060 Bremen (DE)**

(54) **METHOD FOR CONTROLLING AN ELECTROSURGICAL INSTRUMENT CAPABLE OF PRODUCING A CUTTING PLASMA AND CORRESPONDING ELECTROSURGICAL GENERATOR**

(57)     Method for controlling an electrosurgical instrument capable of producing a cutting plasma for cutting or vaporizing tissue when in operation at such tissue, wherein an electrosurgical generator generates a voltage signal being basically sinusoidal and the voltage signal is applied to the electrosurgical instrument, applying the voltage signal to the electrosurgical instrument results in an operating current signal capable of providing the cutting plasma, and the operating current signal comprising a linear current component being sinusoidal having a fundamental frequency and a nonlinear current component not having the fundamental frequency, in particular being non-sinusoidal, and/or being of transient nature, wherein the operating current signal is controlled depending on the nonlinear current component in order to control the cutting plasma.

Fig. 3

## Description

The present invention is directed to a method for controlling an electrosurgical instrument capable of producing a cutting plasma for cutting or vaporizing tissue when in operation at such tissue. The invention is also directed to an electrosurgical generator capable of producing a cutting plasma for cutting or vaporizing tissue.

This invention is related to creating a cutting plasma during a TURis (transurethral resection in saline) procedure. In this procedure, prostate and blattertissue can be cut/vaporized with a plasma ignited around an electrode which is inserted into a so-called working element.

Igniting the plasma involves two steps. First, the saline solution around the electrode is heated with a high current. This creates a gas pocket around the electrode, electrically isolating it from the saline. The high voltage across this gas pocket then causes ionization of the gas and thereby igniting plasma.

Currently, electrosurgical generators used for TURis operate mainly in voltage control. During ignition, the output current is very high as the impedance of the conductive fluid is very low. As soon as a gas pocket has formed, the current automatically reduces as the impedance goes up.

The intensity of the plasma in known systems is controlled via the output voltage. The relation is strongly nonlinear and small changes in the output voltage can significantly change the output power. As an example, there could be three voltage settings used by known system for the different stages and these three voltage settings could be 280 V, 300 V and 320 V.

When there is no tissue contact, it would be beneficial to reduce the voltage as this results in lower output power and less bubbles which obstruct sight. This is challenging in two ways. First, the minimum voltage that is required to maintain a stable plasma condition depends on environmental settings such as temperature, electrode size, electrode position, flow rate, etc.. Second, distinguishing between tissue contact and no-tissue contact is difficult.

A solution could be, to control not on voltage but on current. Direct current control, which involves a current limit, is not feasible however, as the mode requires short bursts of high current whenever the gas layer is about to collapse. Putting a current limit would therefore significantly decrease the stability as the gas layer would become unstable.

Accordingly, the object of the present invention is to address at least one of the above-mentioned problems. In particular, a solution shall be provided, capable of more accurately controlling the cutting plasma and/or more accurately controlling an operating current signal for providing the cutting plasma. At least an alternative solution with respect to solutions known so far shall be provided.

According to the invention, a method is provided according to claim 1. Accordingly, a method is provided for controlling an electrosurgical instrument capable of producing a cutting plasma for cutting or vaporizing tissue when in operation at such tissue, i.e. in the so called working element. According to that method, an electrosurgical generator generates a voltage signal being basically sinusoidal and applying the voltage signal to the electrosurgical instrument, wherein applying the voltage signal to the electrosurgical instrument results in an operating current signal capable of providing the cutting plasma.

Accordingly, there is an electrosurgical generator that basically provides and controls the power supplied to the electrosurgical instrument. This is done such that a voltage signal is applied to the electrosurgical instrument. Such voltage signal is basically sinusoidal. Accordingly, the voltage signal is an alternating signal as a DC signal might harm the patient. The lowest frequency to be used is 200 kHz and accordingly the basically sinusoidal voltage signal has a frequency at 200 kHz or above.

Applying such voltage signal to the electrosurgical instrument results in said operating current. Said operating current is thus controlled by means of the voltage signal applied such that the cutting plasma results.

According to the method, the operating current signal comprises a linear current component which is sinusoidal having a fundamental frequency and a nonlinear current component which is not having the fundamental frequency. In particular, the nonlinear current component may be non-sinusoidal and/or may be of transient nature. However, it might also be possible that the nonlinear current component is sinusoidal, at least has a sinusoidal pat, but not with the fundamental frequency.

Simply speaking, it might be easier to control only a linear and thus sinusoidal current. However, the current providing the cutting plasma changes the impedance which is depending on the cutting plasma. That is of course also depending on the tissue or the surrounding saline and/or the created gas pocket. It was found that this is mainly reflected in the nonlinear current component.

Based on that understanding, it is suggested that the operating current signal is controlled depending on the nonlinear current component in order to control the cutting plasma.

It was found that the process that takes place at or near the tissue and thus at the electrosurgical instrument strongly depends on the nonlinear current component. Both the linear current component and the nonlinear current component are needed to operate the cutting plasma, but only or mainly the nonlinear current component indicates the process that is going on. Accordingly, depending on the nonlinear current the control can be driven such that the cutting plasma is neither getting too strong nor breaking down.

The controlling of the operating current is in particular carried out as a feedback control, such that there is a feedback of the nonlinear current component or a signal indicative of the nonlinear current component. Based on this feedback signal, the operating current is controlled, in particular by means of the applied voltage signal. The feedback signal can be compared with a set value and depending on the comparison the control can be adapted accordingly. However, different feedback control methods can be applied such as a state controller, to give just one further example. The set value may also vary.

According to one aspect, it is suggested that for receiving the nonlinear current component, the linear current component is extracted from the operating current signal such that the nonlinear current component remains.

The linear current component is to be understood as being a sinusoidal signal. Accordingly, such sinusoidal signal and thus the linear current component can easily be described by its amplitude, frequency and phase angle. Accordingly, such linear current component can be described in a simple and also time-invariant manner. At least such shape of the signal does not change too quickly. Such linear current component can be understood as a quasi-stationary signal. At least such linear signal can be understood to have these parameters being constant for at least one period.

Based on that, such linear current component, i.e. such sinusoidal signal being described by these parameters, can thus also quite easily be identified. One possibility is to have a particular filter. It is also possible to use a phase-locked loop (PLL) at least for identifying frequency and phase angle. Other filters can be used as well, such as a Kalman filter.

However, most favourable a Fourier transform, can be used as will be explained further below.

According to one aspect, the linear current component is determined by calculating signal coefficients characterizing a first harmonic of the operating current signal and the linear current component is described using these signal coefficients.

Accordingly, the signal can be analysed using a Fourier transform. Such Fourier transform in general can calculate signal coefficients characterizing first and higher harmonics of the operating current signal. In this way, the operating signal could be described in total using these Fourier signal coefficients.

The underlying idea is that the first harmonic describes the linear component and the higher harmonic components would then describe the nonlinear part of the operating current signal.

However, the signal coefficients for the higher harmonic components do not need to be calculated and instead only the signal coefficients of the first harmonic of the operating current signal are calculated. The result is a clear single sinusoidal current signal.

It was also found that the frequency of the linear current component and thus the frequency of the overall operating current signal is well-known. Accordingly, it is easy to apply the Fourier transform as such transformation needs this frequency of the signal that needs to be transformed. Accordingly, in this way the signal coefficients characterizing the first harmonic of the operating current signal can easily be calculated and thus the linear current component can easily be identified and thus subtracted from the overall operating current signal.

The result from the subtraction is thus the nonlinear current component and this is used for controlling the operating current signal. Accordingly, this is used to be fed back for the feedback control, if such feedback control is used.

According to one aspect, the linear current component is determined by calculating first harmonic Fourier trigonometric coefficients $a_1$ and $b_1$ of the operating current signal according to the formulas:

$$a_1 = \frac{2}{N} \sum_{k=0}^{N-1} x[k] * \cos\left(2\pi \frac{k}{N}\right)$$

and

$$b_1 = \frac{2}{N} \sum_{k=0}^{N-1} x[k] * \sin\left(2\pi \frac{k}{N}\right)$$

wherein

x[k] are samples of the operating current signal and N is the number of samples used, and wherein samples of the operating current signal are taken over a time interval of one period of the linear current signal or the voltage signal.

Accordingly, in this way there is a clear formula for calculating the first harmonic Fourier trigonometric coefficients $a_1$ and $b_1$ which can be understood as the signal coefficients characterizing the first harmonic of the operating current signal.

Accordingly, all that is needed is to sample the operating current signal and calculate these coefficients. For taking these samples, the signal is sampled over a time interval of one period. This period or corresponding frequency is well-known, as the voltage signal is generated by the electrosurgical generator. It is thus also easy to precisely sample over almost exactly a time interval of one period. Accordingly, a good and precise result for the first harmonic Fourier trigonometric coefficients $a_1$ and $b_1$, i.e. the signal coefficients of the first harmonic, can also be calculated with a high quality.

However, it was also found that by this calculation the linear current component can be calculated in a quality that is in fact higher than needed.

Such linear current component can thus just be used to be subtracted from the overall operating current signal resulting in the remaining nonlinear current component which can then be used for controlling the operating current signal.

According to one aspect, the operating current signal is controlled depending on an rms-value of the nonlinear current component and the rms-value of the nonlinear current component is calculated by calculating a square root of a difference between the square root of a rms-value of the operating current signal and the square of an rms-value of the linear current component. Such rms-values are particularly taken over one period of the linear current signal or the voltage signal, or a plurality of such periods, in particular over 2, 3, 4, 5 or more than 5 periods and/or over less than 10. Accordingly, it was found that it is not necessary to consider the instantaneous value but considering the rms-value is enough, even in view of the expected nonlinearities. As a further result, it is also possible to control the operating current by providing a sinusoidal voltage signal, whereas it can be sufficient to only adjust the amplitude of such sinusoidal voltage signal. In other words, it is not necessary to control instantaneous values of this voltage signal.

It was further found that it is enough to calculate the nonlinear current component based on the rms-value of the linear current component and the rms-value of the overall operating current signal.

It was found that in this way the control of the operating current signal is sufficient, whereas the calculation of the nonlinear current component is quite simple as all is needed is an rms-value of the sampled signals and the nonlinear current component defined by said Fourier coefficients.

In particular, even though the operating current signal is controlled depending on the nonlinear current component, such rms-value can be sufficient. It was also found that even though the nonlinear part is characteristic for the operating situation in the working element, an rms-value is enough to consider that operating situation. Considering the particular shape of the nonlinear current component is not necessary. It was also found that the energy used is an important characteristic of the situation and thus the rms value is a good value to describe that.

The sinusoidal voltage signal is adapted depending on the nonlinear current component. In particular, the amplitude of the sinusoidal voltage signal is adjusted depending on rms-value of the nonlinear current component.

According to one aspect, it is suggested that

- for controlling the operating current signal depending on a or the rms-value $I_{nl}$ of the nonlinear current component the rms-value $I_{nl}$ is calculated using the formula:

$$I_{nl} = \sqrt{I_{rms}^2 - \frac{a_1^2 + b_1^2}{2}}$$

wherein

$I_{rms}$ is the rms-value of the operating current signal and

$a_1$ and $b_1$ being the first harmonic Fourier trigonometric coefficients of the operating current signal.

It was thus found that the linear current component can easily be taken into account by using said first harmonic Fourier trigonometric coefficients of the operating current signal. Accordingly, just the rms-value of the sampled current signal and these two coefficients are needed to calculate the nonlinear current component.

In particular, this nonlinear current component can be one value per period of the linear current signal or the voltage signal.

According to one aspect, it is suggested that for controlling the operating current signal the rms-value of the nonlinear current component is controlled to a reference value. The reference value may be understood as a set-value.

It was found that by such reference value for the nonlinear current component a simple and effective control is possible. However, in order to adapt the control to particular situations and/or dynamic behavior in particular of the cutting plasma, the reference value can be constant according to one aspect but can also be variable according to another aspect which is in particular more flexible.

According to one aspect, it is suggested that an amplitude of the voltage signal is increased when the rms-value of the nonlinear current component is below the reference value or is below a first reference value and the amplitude of the voltage signal is decreased when the rms-value of the nonlinear current component is above the reference value or above a second reference value.

Accordingly, the voltage signal can be increased in order to increase the nonlinear current component and it can be decreased in order to decrease the nonlinear current component. The criteria can be the reference value. The underlying concept is the following.

It was found that the nonlinear current component corresponds to the strength or amplitude of the plasma. As long as there is no gas phase, the nonlinear current component is small. Accordingly, in that case, the voltage signal is increased in order to also increase the nonlinear current component. By increasing the amplitude of the voltage signal, a situation will be reached when the high voltage will result in igniting a plasma. As a result, the nonlinear current component will automatically increase significantly. This increase will result in the nonlinear current component exceeding the reference value. Accordingly, the amplitude of the voltage signal will be decreased.

The nonlinear current component will further be observed and used for the controlling. When decreasing the amplitude of the voltage signal, the nonlinear current component will also decrease. The voltage signal will be controlled such that the nonlinear current component will not drop to a too small value as at a too small value the plasma might break down. In that case, the rms-value of the nonlinear current component would be below the reference value and accordingly the amplitude of the voltage signal will be increased again in order to produce more gas phase and thus prevent the breakdown of the plasma.

Accordingly, by the suggested method it is possible to not distinguish between the explained first phase when there is no plasma yet and the second phase when there is plasma. It is to be noted that once the first phase changes into the second phase, i.e. when there is plasma the first time, this switching over from the first to second phase will result in a significant increase of the nonlinear current component. This will also result in the nonlinear current component to exceed the reference value. Simply speaking, the nonlinear current component will jump over this reference value.

For this effect it is not so important which exact value the reference value has. If the reference value is in a reasonable range, the nonlinear current component will increase over this reference value.

However, for avoiding the breakdown of the plasma, and thus for controlling the plasma in the second phase, the reference value can be chosen such that the plasma will not break down as long as the nonlinear current component does not fall significantly below the reference value. Accordingly, the reference value can be chosen depending on such value that is considered to be a safe value for avoiding the breakdown of the plasma. Such reference value will also work for the first phase where the nonlinear current component is low and thus below the reference value as long as there is no plasma ignited.

Such reference value can be found by making tests. Such tests can be done by controlling the operating current in a traditional manner and observing the nonlinear current component. Such reference value can also be found by using a variable reference value that is adapted during such operation process. It is also possible to make tests in an environment that is similar to that environment which will finally be present when operating on real tissue. Simulations are also possible to find the adequate reference value.

However, it is also possible to use different reference values, i.e. a first reference value for controlling the first phase, i.e. for controlling the situation when the nonlinear current component is below such first reference value. For the second phase, the second reference value may be used, i.e. to control the nonlinear current component such that the plasma does not break down. Changing over from the first to the second reference value could take place once the nonlinear current component exceeds the first reference value. However, it is also possible to adapt a single reference value by continuously varying it.

The reference value or the first and second reference value may each be predetermined or may vary. One possibility on how to vary these reference values is to change or adapt these during control. Such control could be an adaptive control that might change the reference values depending on identified changes of the environment in which the electrosurgical instrument is operating.

According to one aspect, it is suggested that in a first control stage for heating a saline, the rms-value of the nonlinear current component is controlled to a or the first reference value and in a second control stage for providing the current cutting plasma the rms-value of the nonlinear current component is controlled to a second reference value, wherein in particular the first reference value is larger than the second reference value.

Accordingly, this aspect is similar to the previous aspects, whereas according to this aspect the first and second control stages are more clearly identified. It was found that the first reference value shall be larger than the second reference value as in the first control stage, which relates to the above explained first phase, the voltage signal first needs to be increased such that also the nonlinear current component is increased such that the plasma is ignited. Once the plasma is ignited, the control goes over to the second control stage and the nonlinear current component may decrease again. In other words, for igniting the plasma, a higher current is needed than for controlling the plasma which already exists.

According to one aspect, it is suggested that the electrosurgical instrument comprises a single electrode for providing the cutting plasma between the electrode and a saline or a neighboring tissue. Accordingly, the control of the cutting plasma depends on the interaction between the electrode and the saline or between the electrode and the neighboring tissue. However, the suggested control depending on the nonlinear current component provides a very fast control, as the voltage signal can be adjusted each period of the linear current signal or the voltage signal.

In other words, as the frequency of the operating current signal is at 200 kHz or above, that can also be the frequency of the control cycle. Accordingly, any change in the interaction between the electrode and the saline or the neighboring tissue will result in a change in the nonlinear current component and accordingly will be considered by the control. Thus, the operating current signal will be adapted quite quickly to compensate for any changes in the interaction between the electrode and the saline or the neighboring tissue.

It is also possible that the electrosurgical instrument comprises two electrodes for providing the cutting plasma between these two electrodes. Any change of the saline or tissue between these two electrodes will have influence on the plasma and thus the operating current signal. However, it will also have influence on the nonlinear current component and that will be considered by the control as explained for the single electrode as well.

According to the invention, there is also provided an electrosurgical generator for controlling an electrosurgical instrument capable of producing a cutting plasma for cutting or vaporizing tissue when in operation at such tissue, wherein the electrosurgical generator is adapted for executing a method according to which

- the electrosurgical generator generates a voltage signal being basically sinusoidal and the voltage signal is applied to the electrosurgical instrument, wherein

- applying the voltage signal to the electrosurgical instrument results in an operating current signal capable of providing the cutting plasma, and

- the operating current signal comprising

- a linear current component being sinusoidal having a fundamental frequency and

  - a nonlinear current component not having the fundamental frequency, and in particular being non-sinusoidal and/or being of transient nature, wherein

  - the operating current signal is controlled depending on the nonlinear current component in order to control the cutting plasma.

In particular, such electrosurgical generator is adapted to execute a method as explained above. The used electrosurgical instrument may be designed as explained above. The electrosurgical generator can be adapted such that the method, in particular a corresponding program, is implemented on the electrosurgical generator.

According to one aspect, it is suggested that the electrosurgical generator comprises a control device adapted to control the electrosurgical generator, wherein the electrosurgical generator and in particular the control device is adapted to execute a method according to any of the above explained aspects.

Accordingly, there is provided a control device which can be a processor adapted to control a frequency converter. The frequency converter may be part of the electrosurgical generator and may be capable of generating a voltage signal, in particular, a basically sinusoidal voltage signal. The method may thus be implemented on such processor and the processor is adapted to control the frequency converter and the frequency converter is adapted to provide the voltage signal as an output voltage signal to an electrosurgical instrument connected to the electrosurgical generator.

According to one aspect, the electrosurgical generator comprises a frequency converter for providing the voltage signal being coupled to the control device and/or comprising an output port for connecting an electrosurgical instrument capable of producing a cutting plasma for cutting or vaporizing tissue when controlled by the generator and when in operation at such tissue and/or comprising a current sensor for measuring an operational current and being coupled to the frequency converter or the control device.

Accordingly, the frequency converter may provide the voltage signal and the frequency converter is thus coupled to the control device. Accordingly, the control device can control the frequency converter and thereby the voltage signal.

The electrosurgical instrument may be coupled to the output port and in this way the voltage signal generated by the frequency converter is provided via this output port to a connected electrosurgical instrument.

In this way, the electrosurgical instrument is controlled by the frequency converter which is controlled by the control device.

The generator may comprise the current sensor for measuring an operational current. Such operational current is the result of providing the voltage signal to the electrosurgical instrument when in operation in or at a tissue, i.e. when in operation at a working element. The current sensor is thus coupled to the frequency converter or the control device or both. This current sensor can thus be used to sample the operational current and the sampled values can be further used for calculating a linear current component, in particular first harmonic Fourier trigonometric coefficients based on these sampled values. Based on that, the sampled values can further be used to calculate the nonlinear current component as explained above.

According to the invention there is provided an electrosurgical installation comprising an electrosurgical generator according to any of above explained aspects, and comprising an electrosurgical instrument connected to the electrosurgical generator.

Such electrosurgical instrument may thus be designed as explained above.

The invention is now explained by way of example based on the accompanying figures.

Figure 1    shows an electrosurgical generator.

Figure 2    shows a simplified control structure.

Figure 3    shows voltage and current signals.

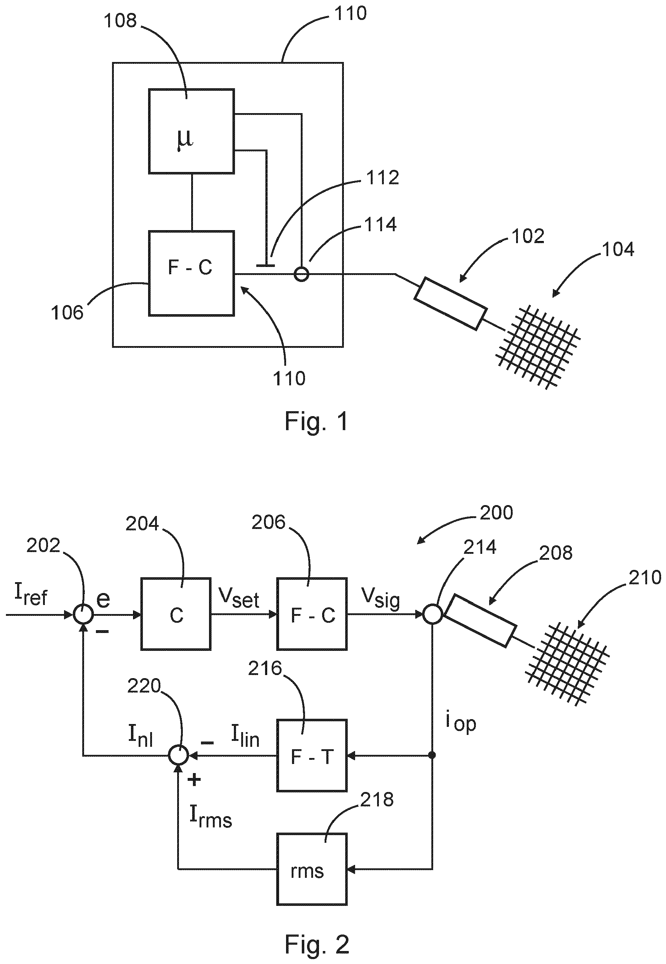

Figure 1 shows an electrosurgical generator 100 to which an electrosurgical instrument 102 is connected operating on a tissue 104, i.e. a working element, just schematically illustrated.

The electrosurgical generator 100 comprises a frequency converter 106 which is controlled by a control device 108. The frequency converter 106 when in operation provides at its output 110 a voltage signal which is applied to the attached electrosurgical instrument 102, resulting in an operational current.

There is a voltage sensor 112 for measuring the voltage signal and a current sensor 114 for measuring the operational current. These sensors 112, 114 are connected to the control device 108 for further consideration.

The control device 108 calculates from the current sample values provided by the current sensor 114 a nonlinear current component. Based on that, the control device 108 controls the frequency converter 106. Accordingly, at the frequency converter output, the voltage signal is generated depending on the calculated nonlinear current component.

The voltage sensor 112 might not be necessary as it is known what kind of voltage the voltage converter 106 generates. It is also possible that the voltage sensor 112 is connected to the frequency converter 106 directly. It is also possible that the control device 108 is part of the frequency converter 106.

Figure 2 shows a schematic control structure 200. This control structure 200 illustrates that the control is based on a nonlinear current component $I_{nl}$ and a reference current $I_{ref}$. These values are subtracted in the first summing element 202 resulting in a control error e. This control error e is passed to the control block 204 resulting in a set value for the voltage signal $V_{set}$. This set value for the voltage signal $V_{set}$ is given to the frequency converter 206 which could be identical to the frequency converter 106 according to Figure 1.

The result of the frequency converter 206 is the voltage signal $V_{sig}$. This voltage signal is passed to the electrosurgical instrument 208 which could be identical to the electrosurgical instrument 102 according to Figure 1. The electrosurgical instrument 208 is also operating on a schematically illustrated tissue 210, i.e. on a working element.

Applying the voltage signal $V_{sig}$ to the electrosurgical instrument 208 results in an operational current $i_{OP}$. This operational current $i_{OP}$ is measured by means of a current sensor 214. This operational current $i_{OP}$ is thus the sampled current sampled by the current sensor 214. This sampled current is given to the Fourier transform block 216 and to the rms-block 218. The Fourier transform block 216 calculates first harmonic Fourier trigonometric coefficients and in this way identifies the linear current component $I_{lin}$. The rms-block 218 calculates the root mean square value $I_{rms}$ and thus the effective value of the sampled current $i_{OP}$. In the second summing element 220, a difference of the rms-value of the operational current $I_{rms}$ and the linear current component $I_{lin}$ is calculated resulting in the nonlinear current component $I_{nl}$. Accordingly, the nonlinear current component $I_{nl}$ is also an rms-value. However, this calculation of the nonlinear current component $I_{nl}$ is just a simplified illustration in Figure 2 not showing all calculation steps and preferably the current component $I_{nl}$ is calculated by taking the root of the difference of the square of the rms-value of the

operating current $I_{rms}$ and the square of the linear current component $I_{lin}$. I.e. the second summing element 220 is a simplified illustration of $I_{nl}=\text{sqrt}(I_{rms}{}^2-I_{lin}{}^2)$.

However, in this way, the nonlinear current component $I_{nl}$ is calculated and provided to the first summing element 202 as explained above.

The control block 204 can be designed such that depending on the error signal e, i.e. the result of the comparison of the reference value $I_{ref}$ and the nonlinear current component $I_{nl}$, the voltage set value $V_{set}$ is either increased or decreased. The behavior might be similar to a PI-control. However, preferably, this can also be realized in a digital manner such that in each control cycle the voltage set value can be increased by a certain increment, if the error signal e is positive, or it can be decreased by a particular increment, which can be the same as for the explained increase, when the error signal is negative. Accordingly, this can be different to a I-behavior, as according to one aspect the amplitude of the error signal might not be taken into account, but only whether it is negative or positive. However, considering the particular value of the error signal might also be an option.

It is to be noted that the possibility of increasing the voltage set value by a constant increment per control cycle will result in almost a continuous behavior as the cycle frequency can be at 200 kHz or above.

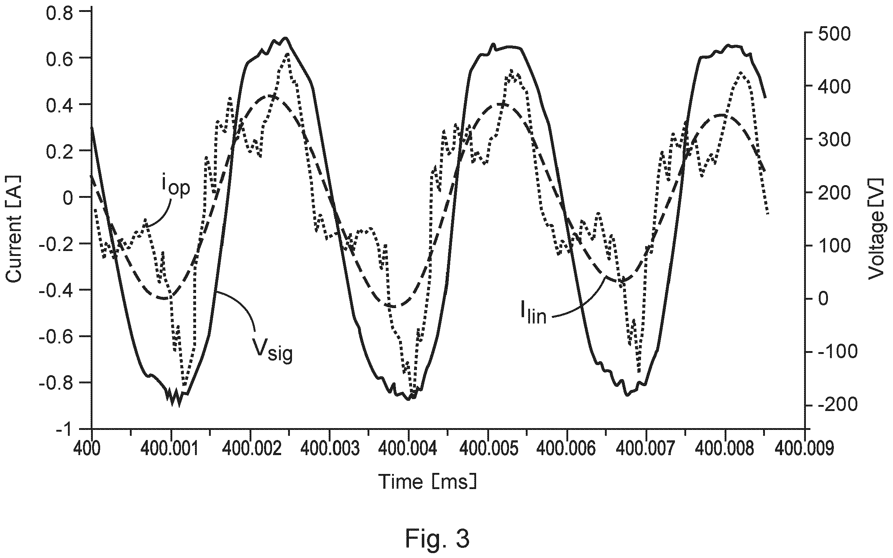

Figure 3 shows recorded voltage and current data during TURis operation. The voltage is approximately sinusoidal while the current consists of a linear part and nonlinear part corresponding to plasma breakthroughs. The nonlinear current component can also be understood as "plasma current" and cannot be measured directly. The idea is therefore to separate the linear current component from the nonlinear current component, i.e. from the "plasma current' and control on the "plasma current" only.

One way to separate the currents is to calculate the first harmonic of the measured current using the Fourier transform. This includes any capacitive and ohmic currents. Subtracting this measured current from the totally measured current yields an estimate of the nonlinear current component, i.e. of the "plasma current" which can then be used for control. Since only the rms-value of the "plasma current" is relevant, it can be calculated from the rms-value of the total current and the calculated Fourier coefficients $a_1$ and $b_1$ according to:

$$l_{nl} = \sqrt{l_{rms}{}^2 - l_{lin}{}^2} = \sqrt{l_{rms}{}^2 - \frac{a_1{}^2 + b_1{}^2}{2}}$$

Figure 3: The solid lines show measured voltage $V_{sig}$ and current $i_{op}$ data during TURis operation of an electrosurgical generator. The current is a combination of a capacitive current, which is 90° phase shifted with respect to the voltage, and current peaks which are in phase with the voltage. During ignition, there is a very high current in phase with the voltage. The dashed line shows the first harmonic component $I_{lin}$ of the sampled current $i_{OP}$.

**Claims**

1.  Method for controlling an electrosurgical instrument capable of producing a cutting plasma for cutting or vaporizing tissue when in operation at such tissue, wherein

    - an electrosurgical generator generates a voltage signal being basically sinusoidal and the voltage signal is applied to the electrosurgical instrument,
    - applying the voltage signal to the electrosurgical instrument results in an operating current signal capable of providing the cutting plasma, and
    - the operating current signal comprising

        - a linear current component being sinusoidal having a fundamental frequency and
        - a nonlinear current component not having the fundamental frequency, in particular being non-sinusoidal, and/or being of transient nature, wherein

    - the operating current signal is controlled depending on the nonlinear current component in order to control the cutting plasma.

2.  Method according to claim 1, **characterized in that**

    - for receiving the nonlinear current component, the linear current component is extracted from the operating current signal such that the nonlinear current component remains.

3.  Method according to claims 1 or 2, **characterized in that**

    - the linear current component is determined by calculating signal coefficients characterizing a first harmonic of the operating current signal and
    - the linear current component is described using these signal coefficients.

4.  Method according to any of the preceding claims, **characterized in that**

    - the linear current component is determined by calculating first harmonic Fourier trigonometric coefficients $a_1$ and $b_1$ of the operating current signal according to the formulas:

$$a_1 = \frac{2}{N} \sum_{k=0}^{N-1} x[k] * \cos\left(2\pi \frac{k}{N}\right)$$

and

$$b_1 = \frac{2}{N} \sum_{k=0}^{N-1} x[k] * \sin\left(2\pi \frac{k}{N}\right)$$

wherein

$x[k]$ are samples of the operating current signal and
N is the number of samples used, and wherein
samples of the operating current signal are taken over a time interval of one period of the linear current signal or the voltage signal.

5.  Method according to any of the preceding claims, **characterized in that**

    - the operating current signal is controlled depending on an rms-value of the nonlinear current component and
    - the rms-value of the nonlinear current component is calculated by calculating a square root of a difference between the square of a rms-value of the operating current signal and the square of an rms-value of the linear current component.

6.  Method according to any of the preceding claims, **characterized in that**

    - for controlling the operating current signal depending on a or the rms-value $I_{nl}$ of the nonlinear current component the rms-value $I_{nl}$ is calculated using the formula:

$$I_{nl} = \sqrt{I_{rms}^2 - \frac{a_1^2 + b_1^2}{2}}$$

wherein

$I_{rms}$ is the rms-value of the operating current signal and
$a_1$ and $b_1$ being the first harmonic Fourier trigonometric coefficients of the operating current signal.

7.  Method according to any of the preceding claims, **characterized in that**

- for controlling the operating current signal a or the rms-value of the nonlinear current component is controlled to a reference value, and/or
- an amplitude of the voltage signal is increased when the rms-value of the nonlinear current component is below the reference value or below a first reference value and
- the amplitude of the voltage signal is decreased when the rms-value of the nonlinear current component is above the reference value or above a second reference value.

8. Method according to any of the preceding claims, **characterized in that**

- in a first control stage for heating a saline, a or the rms-value of the nonlinear current component is controlled to a or the first reference value and
- in a second control stage for providing the cutting plasma the rms-value of the nonlinear current component is controlled to a second reference value, wherein
- the first reference value is larger than the second reference value.

9. Method according to any of the preceding claims, **characterized in that**

- the electrosurgical instrument comprises a single electrode for providing the cutting plasma between the electrode and a saline or a neighboring tissue or
- the electrosurgical instrument comprises two electrodes for providing the cutting plasma between these two electrodes.

10. Electrosurgical generator for controlling an electrosurgical instrument capable of producing a cutting plasma for cutting or vaporizing tissue when in operation at such tissue, wherein the electrosurgical generator is adapted for executing a method according to which

- the electrosurgical generator generates a voltage signal being basically sinusoidal and the voltage signal is applied to the electrosurgical instrument, wherein
- applying the voltage signal to the electrosurgical instrument results in an operating current signal capable of providing the cutting plasma, and
- the operating current signal comprising

- a linear current component being sinusoidal having a fundamental frequency and
- a nonlinear current component not having the fundamental frequency, in particular being non-sinusoidal, and/or being of transient nature, wherein

- the operating current signal is controlled depending on the nonlinear current component in order to control the cutting plasma.

11. Electrosurgical generator according to claim 10, comprising

- a control device adapted to control the electrosurgical generator, wherein
- the electrosurgical generator and in particular the control device is adapted to execute a method according to any of claims 1 to 9.

12. Electrosurgical generator according to claim 10 or 11, comprising

- a frequency converter for generating the voltage signal, being coupled to a or the control device, and/or
- an output port for connecting an electrosurgical instrument capable of producing a cutting plasma for cutting or vaporizing tissue when controlled by the generator and when in operation at such tissue and/or
- a current sensor for measuring an operational current and being coupled to the frequency converter and/or the control device.

13. Electrosurgical installation comprising

- an electrosurgical generator according to any of claims 10 to 12, and
- an electrosurgical instrument connected to the electrosurgical generator.

Fig. 1

Fig. 2

Fig. 3

EP 4 477 169 A1

Europäisches
Patentamt

European
Patent Office

Office européen
des brevets

## EUROPEAN SEARCH REPORT

**Application Number**

EP 24 18 0700

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/198217 A1 (STRAUSS TIMO [DE] ET AL) 5 August 2010 (2010-08-05) * paragraphs [0001], [0002], [0025], [0041], [0053], [0054]; figures 6-8 * | 1-13 | INV. A61B18/12 |
| X | US 2008/208185 A1 (FISCHER KLAUS [DE] ET AL) 28 August 2008 (2008-08-28) * paragraphs [0008], [0017], [0032], [0042]; figures 1-4 * | 1-13 | |
| X | EP 0 219 568 A1 (ERBE ELEKTROMEDIZIN [DE]) 29 April 1987 (1987-04-29) * the whole document * | 1-11,13 | |
| X | US 2003/158546 A1 (FARIN GUNTER [DE] ET AL) 21 August 2003 (2003-08-21) * paragraphs [0014], [0024]; figure 5 * | 1-11,13 | |
| A | US 2016/113702 A1 (KELLER SANDRA [DE]) 28 April 2016 (2016-04-28) * paragraphs [0015], [0036] - [0042]; figures 1, 3 * | 1-13 | |
| A | US 2022/395310 A1 (MARION DUANE W [US] ET AL) 15 December 2022 (2022-12-15) * paragraphs [0022], [0023], [0047]; figure 3 * | 1-13 | TECHNICAL FIELDS SEARCHED (IPC) A61B |
| A | US 4 860 745 A (FARIN GUENTER [DE] ET AL) 29 August 1989 (1989-08-29) * column 15, line 24 - column 16, line 30; figure 1 * | 1-13 | |

The present search report has been drawn up for all claims

| Place of search | Date of completion of the search | Examiner |
|---|---|---|
| Munich | 21 October 2024 | Lorenz, Larissa |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 0700

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2024

| Patent document cited in search report | | Publication date | Patent family member(s) | | Publication date |
|---|---|---|---|---|---|
| US 2010198217 | A1 | 05-08-2010 | DE | 102007034271 A1 | 22-01-2009 |
| | | | EP | 2170201 A1 | 07-04-2010 |
| | | | EP | 2491882 A2 | 29-08-2012 |
| | | | JP | 5538218 B2 | 02-07-2014 |
| | | | JP | 2010533528 A | 28-10-2010 |
| | | | US | 2010198217 A1 | 05-08-2010 |
| | | | WO | 2009010565 A1 | 22-01-2009 |
| US 2008208185 | A1 | 28-08-2008 | CN | 101056592 A | 17-10-2007 |
| | | | DE | 102004054575 A1 | 24-05-2006 |
| | | | EP | 1816969 A1 | 15-08-2007 |
| | | | JP | 4842959 B2 | 21-12-2011 |
| | | | JP | 2008519620 A | 12-06-2008 |
| | | | US | 2008208185 A1 | 28-08-2008 |
| | | | WO | 2006050888 A1 | 18-05-2006 |
| EP 0219568 | A1 | 29-04-1987 | EP | 0219568 A1 | 29-04-1987 |
| | | | JP | H0586226 B2 | 10-12-1993 |
| | | | JP | S62101239 A | 11-05-1987 |
| US 2003158546 | A1 | 21-08-2003 | NONE | | |
| US 2016113702 | A1 | 28-04-2016 | BR | 102015021518 A2 | 26-04-2016 |
| | | | CN | 105534594 A | 04-05-2016 |
| | | | EP | 3011923 A1 | 27-04-2016 |
| | | | JP | 6152153 B2 | 21-06-2017 |
| | | | JP | 2016085210 A | 19-05-2016 |
| | | | KR | 20160047979 A | 03-05-2016 |
| | | | PL | 3011923 T3 | 14-02-2022 |
| | | | US | 2016113702 A1 | 28-04-2016 |
| US 2022395310 | A1 | 15-12-2022 | NONE | | |
| US 4860745 | A | 29-08-1989 | EP | 0253012 A1 | 20-01-1988 |
| | | | EP | 0430929 A2 | 05-06-1991 |
| | | | JP | H0761340 B2 | 05-07-1995 |
| | | | JP | S6324933 A | 02-02-1988 |
| | | | US | 4860745 A | 29-08-1989 |

EPO FORM P0459

For more details about this annex : see Official Journal of the European Patent Office, No. 12/82